# EUROPEAN PATENT SPECIFICATION

(11) **EP 3 914 050 B1**
(45) Date of publication and mention of the grant of the patent: **02.08.2023**
(21) Application number: 20196982.1
(22) Date of filing: 18.09.2020
(51) Int. Cl.: H05H 1/24, A01G 5/06, A01N 3/02, A23B 7/015, A61L 2/14

(54) **SYSTEM AND METHOD FOR PRESERVING CUT-FLOWERS USING AN ATMOSPHERIC PRESSURE PLASMA GENERATOR**
SYSTEM UND VERFAHREN ZUR KONSERVIERUNG VON SCHNITTBLUMEN MITTELS EINES ATMOSPHÄRENDRUCKPLASMAGENERATORS
SYSTÈME ET PROCÉDÉ DE CONSERVATION DE FLEURS COUPÉES AVEC UN GÉNÉRATEUR DE PLASMA À PRESSION ATMOSPHÉRIQUE

(30) Priority: 22.05.2020 KR 20200061333; 16.09.2020 KR 20200119506
(43) Date of publication of application: 24.11.2021
(73) Proprietor: Lee, Chang Hoon, Seoul 03477 (KR)
(72) Inventor: Lee, Chang Hoon, Seoul 03477 (KR)
(74) Representative: Studio Torta S.p.A.

(56) References cited:
- CN-U- 202 168 366
- JP-A- H0 912 403
- JP-A- H07 165 503
- JP-A- 2000 300 173
- JP-A- 2016 214 207
- KR-A- 20160 062 876
- KR-A- 20190 066 214

## Description

### BACKGROUND

### Technical field

The present disclosure relates to a system and method for preserving cut-flowers, and more specifically, to a system and method for preserving cut-flowers using an atmospheric pressure plasma generator.

### Description of the Related Art

Plasma is an ionized gas such as electrons, neutral particles, or the like, and a part of the plasma gas has high energy, which can change a surface of a material. That is, the plasma may react directly with the surface of another material or react by elastic collision. The plasma generator mainly includes a tube which is configured to generate plasma with the compressed air or nitrogen gas colliding with high-frequency, high-voltage electric charges. In recent years, the use of atmospheric pressure plasma devices in place of low pressure or vacuum plasma is increasing. The atmospheric pressure plasma device may be applied to various materials and substrates through a low-temperature process, and since a vacuum container or a vacuum evacuation device is not necessary, the treatment is fast and economical. In addition, when used, a deposition method using the atmospheric pressure plasma provides good adhesion and low deposition temperature, and thus, it is used in relatively various industries by utilizing the advantages such as reduction in deformation or denaturation that accompanies high temperature heating in the conventional surface treatment process, semiconductor process, and display process.

In general, ethylene oxide (EO) gas, ozone, low-temperature plasma, and the like may be used for disinfection and sterilization of biomaterials such as medical equipment or food. Among them, the medical sterilizer using plasma gas uses a hydrogen peroxide sterilization method, but there is a problem that a large amount of cost and space are required to purchase and operate the equipment. Meanwhile, the atmospheric pressure plasma generator generates, by discharge, various active species including ozone. Since these active species have a sterilization, disinfection or bleaching action, they can be used for sterilization or disinfection or air purification of various articles or biomaterials. Known systems are disclosed in published documents JPH07165503 A, KR20160062876 A, KR20190066214 A, JP200300173 A, CN202168366 U, JP2016214207 A or JPH0912403 A.

### SUMMARY

Embodiments disclosed herein provide a system and method for preserving cut-flowers, capable of controlling moisture and of antibacterial treatment inside a container for preserving cut-flowers, using a plasma generator and an air filter that generates compressed air.

In particular, it is provided a system and a method for preserving cut-flowers as claimed in the appended set of claims.

According to the claimed system and method, it is possible to increase the freshness and preservation period of cut-flowers by removing moisture contained in air in a container in which cut-flowers are preserved to maintain a constant humidity.

In addition by generating a plasma gas having a sterilization or disinfection effect while maintaining a constant humidity of the air in the container in which the cut-flowers are preserved, the freshness and preservation period of the cut-flowers can be further increased, thereby improving the marketability of the cut-flowers.

Further , by including a preservative in a cut-flower receptacle made of an antibacterial material, antibacterial and disinfection of the preservative are enabled and it is possible to increase the freshness and preservation period of the cut-flower.

The effects of the present disclosure are not limited to the effects described above, and other effects that are not mentioned above can be clearly understood to those skilled in the art based on the description provided below.

### DESCRIPTION OF THE DRAWINGS

The above and other objects, features and advantages of the present disclosure will become more apparent to those of ordinary skill in the art by describing in detail exemplary embodiments thereof with reference to the accompanying drawings, in which:
- FIG. 1 is a schematic diagram showing a system for preserving cut-flowers using an atmospheric pressure plasma generator according to an embodiment of the present disclosure;
- FIG. 2 is a schematic diagram showing a system for preserving cut-flowers using an atmospheric pressure plasma generator according to another embodiment of the present disclosure;
- FIG. 3 is a perspective view schematically showing a plasma generator according to an embodiment of the present disclosure;

- FIG. 4 is a cross-sectional view of a tray for preserving cut-flowers according to an embodiment of the present disclosure;
- FIG. 5 is a flowchart showing a method for preserving cut-flowers using an atmospheric pressure plasma generator according to an embodiment of the present disclosure; and
- FIG. 6 is a flowchart showing a method for preserving cut-flowers using an atmospheric pressure plasma generator according to another embodiment of the present disclosure.

### DETAILED DESCRIPTION

Hereinafter, specific details for the practice of the present disclosure will be described in detail with reference to the accompanying drawings. However, in the following description, detailed descriptions of well-known functions or configurations will be omitted when it may make the subject matter of the present disclosure rather unclear.

In the accompanying drawings, the same or corresponding components are given the same reference numerals. In addition, in the following description of the embodiments, duplicate descriptions of the same or corresponding components may be omitted. However, even if descriptions of components are omitted, it is not intended that such components are not included in any embodiment.

The terms used in the present disclosure will be briefly described prior to describing the disclosed embodiments in detail. The terms used herein have been selected as general terms which are widely used at present in consideration of the functions of the present disclosure, and this may be altered according to the intent of an operator skilled in the art, conventional practice, or introduction of new technology. In addition, in a specific case, a term is arbitrarily selected by the applicant, and the meaning of the term will be described in detail in a corresponding description of the embodiments. Therefore, the terms used in the present disclosure should be defined based on the meaning of the terms and the overall contents of the present disclosure rather than a simple name of each of the terms.

In the present disclosure, the singular forms "a," "an," and "the" are intended to include the plural forms as well, unless the context clearly indicates the singular forms. Further, the plural forms are intended to include the singular forms as well, unless the context clearly indicates the plural forms.

In the present disclosure, when a portion is stated as "comprising (including)" a component, unless specified to the contrary, it intends to mean that the portion may additionally comprise (or include or have) another component, rather than excluding the same.

As used herein, the statement "A and/or B" means A, or B, or A and B.

Prior to describing the embodiments of the present disclosure, it is to be noted that the upper direction of the drawing may be referred to as "upper portion" or "upper side" of the configuration shown in the drawing, and the lower direction may be referred to as "lower portion" or "lower side". In addition, in the drawings, a portion between the upper and lower portions of the configuration shown in the drawings, or a portion other than the upper and lower portions may be referred to as "side portion" or "side". Relative terms such as "upper portion" and "upper side" may be used to describe the relationship between components shown in the drawings, and the present disclosure is not limited by these terms.

In embodiments of the present disclosure, the left direction of the drawing may be referred to as "left" or "left side" of the configuration shown in the drawing, and the right direction thereof may be referred to as "right" or "right side". Relative terms such as "left" and "left side" may be used to describe the relationship between components shown in the drawings, and the present disclosure is not limited by these terms.

In the present disclosure, a direction toward the inner space of a structure may be referred to as "inside", and a direction projecting into the open outer space may be referred to as "outside". Relative terms such as "inside" and "outside" may be used to describe the relationship between components shown in the drawings, and the present disclosure is not limited by these terms.

Throughout the description, when a portion is stated as being connected to another portion, it intends to include not only an example in which the portions are directly connected, but also an example in which the portions are connected while having another component disposed therebetween.

In the present disclosure, the term "part" or "portion" or "module" means a mechanical or hardware component, a software component, or a combination thereof, and a "unit" or "module" may be configured to perform a specific role or function. However, it does not mean that "unit" or "module" is limited to a mechanical component or hardware or software. The "unit" or "module" may be configured to be in an addressable storage medium or to execute one or more processors. Accordingly, as an example, the "unit" or "module" includes elements such as software elements, object-oriented software elements, class elements, and task elements, processes, functions, attributes, procedures, subroutines, program code segments, drivers, firmware, micro-codes, circuits, data, database, data structures, tables, arrays, and variables. Functions provided in the components and the "units" or "modules" described in the present disclosure may be combined as a smaller number of components and "units" or "modules", or further divided into additional components and "units" or "modules".

In the present disclosure, "system" may mean a mechanical device or an electromechanical device including one or more plasma generators, air compressors, air filters, computing devices, containers, and the like, but is not limited thereto.

Advantages and features of the disclosed embodiments and methods of accomplishing the same will be apparent by referring to embodiments described below in connection with the accompanying drawings. However, the present disclosure is not limited to the embodiments disclosed below, and may be implemented in various different forms, and the present embodiments are merely provided to make the present disclosure complete, and to fully disclose the scope of the invention to those skilled in the art to which the present disclosure pertains.

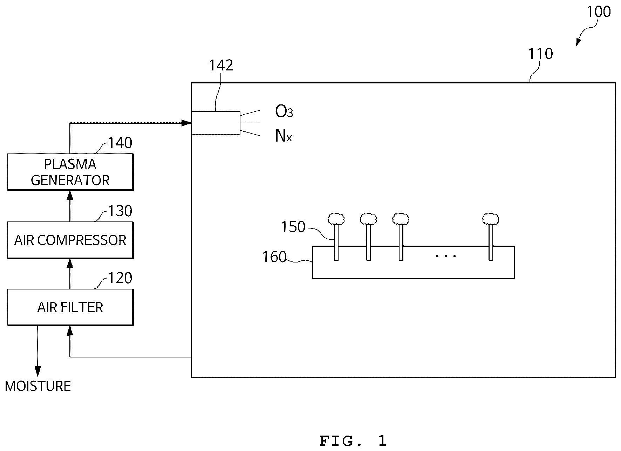

FIG. 1 is a schematic diagram showing a system 100 for preserving cut-flowers using an atmospheric pressure plasma generator according to an embodiment of the present disclosure.

As shown, the system 100 for preserving cut-flowers includes a container 110 in which one or more cut-flowers 150 are preserved, an air filter 120 that extracts at least some of moisture contained in air in the container 110 and discharges the same, an air compressor 130 that generates compressed air from the air from which at least some of the moisture was removed by the air filter 120, and a plasma generator 140 that generates active species through plasma discharge using the compressed air generated from the air compressor 130 as a plasma discharge gas.

The container 110 is used for the purpose of preservation and may be used for the transport of the cut-flowers 150 for a certain period of time until the cut-flowers 150 from the production site are delivered to the purchaser. Accordingly, the container 110 may be installed in a fixed position or configured to be detachable from transport equipment such as a vehicle or a ship.

In addition, one or more supports 160 for the cut-flowers 150 to be mounted thereon may be disposed in the container 110. The support 160 may be configured to mount and preserve the cut-flowers 150. In an embodiment, the support 160 may be in a box shape with an empty space inside, and may include a plurality of through holes through which the cut-flowers 150 can be inserted into the support 160.

The system 100 for preserving cut-flowers may be configured such that an air filter 120 is connected to one side of the container 110, thus allowing air in the container 110 to be introduced into the air filter 120. The air filter 120 is configured to remove at least some of the moisture contained in the air introduced from the container 110. With this configuration, the air in the container 110 may be managed at a constant humidity. In an embodiment, the air filter 120 may include a cooling or dry dehumidifier. For example, the dry dehumidifier may be configured to adsorb the moisture contained in the air by using a desiccant such as silica gel, alumina gel, or molecular sieves, which are porous materials. In another example, the cooling dehumidifier may be configured to suck in air with a fan and then pass it through a cooling device where refrigerant is used, thereby using a characteristic of the air that turns into water as the temperature of the air is decreased to the dew point.

The air, from which moisture is removed by the air filter 120, is introduced into the air compressor 130. The air compressor 130 compresses the introduced air to a constant pressure and provide it to the plasma generator 140. In an embodiment, the air compressor 130 may include a reciprocating or piston type, a rotary screw type, or a turbo or centrifugal type compressor, but is not limited thereto, and may include any type of compressor that has an appropriate capacity to provide air of a constant pressure as required by the plasma generator 140.

The plasma generator 140 generates active species including ozone (O₃) through plasma discharge using the introduced compressed air as the plasma discharge gas. In an embodiment, the plasma generator 140 may be an atmospheric pressure plasma generator. The plasma gas including the active species generated by the plasma generator 140 may be discharged into the container through a nozzle part 142 attached inside the container 110. The harmful bacteria or harmful substances contained in the air inside the container 110 may be removed by the plasma gas including the active species discharged as described above, and accordingly, the freshness or preservation period of the cut-flowers 150 may be increased.

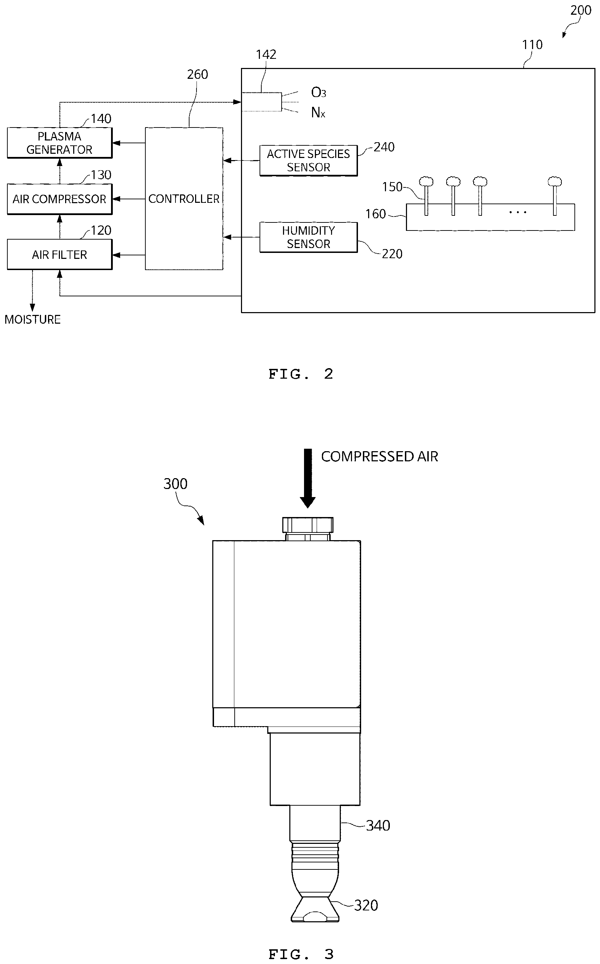

FIG. 2 is a schematic diagram showing a system 200 for preserving cut-flowers using an atmospheric pressure plasma generator according to another embodiment of the present disclosure. Among the configurations disclosed in FIG. 2, a description of the configurations corresponding to those of FIG. 1 will be omitted.

As shown, the system 200 for preserving cut-flowers includes the container 110, the air filter 120, the air compressor 130, the plasma generator 140, a humidity sensor 220 and an active species sensor 240 installed in the container 110, and a controller 260 that controls one or more of the air filter 120, the air compressor 130, and the plasma generator 140 so that the air in the container 110 has a constant humidity and concentration of active species. In the system 200 for preserving cut-flowers, the controller 260 that received a signal from the humidity sensor 220 and/or the active species sensor 240 controls the air filter 120, the air compressor 130, and/or the plasma generator 140, thereby maintaining the humidity and the concentration of active species of the air inside the container 110 within a predetermined numerical range.

In an embodiment, the humidity sensor 220 may be installed inside the container 110. The humidity sensor 220 installed inside the container 110 may be configured to measure the amount of moisture or humidity of the air inside the container 110. Examples of the humidity sensor 220 may include an electric resistive type or electric capacitive type humidity sensor capable of outputting an electrical signal according to humidity, but it is not limited thereto. The humidity sensor 220 may serve to check, in real time, whether or not the amount of moisture or humidity of the air inside the container 110 is maintained within the predetermined numerical range. The humidity sensor 220 may transmit, to the controller 260, an electrical signal determined according to the amount of moisture or humidity contained in the air inside the container 110.

In an embodiment, the active species sensor 240 may be installed inside the container 110. The active species sensor 240 installed inside the container 110 may be configured to measure the concentration of the active species (e.g., O₃, Nₓ, and the like) of the air inside the container 110. Examples of the active species sensor 240 may include a chemical sensor capable of outputting an electrical signal by changing electrical conductivity according to adsorption or desorption of active species in gaseous state, but it is not limited thereto. The active species sensor 240 may serve to check, in real time, whether or not the concentration of active species of the air inside the container 110 is maintained at a concentration equal to or higher than a reference value. The active species sensor 240 may transmit, to the controller 260, an electrical signal determined according to the presence or absence of the active species contained in the air inside the container 110 or the concentration of the active species.

The controller 260 may receive an electrical signal transmitted from the humidity sensor 220 and/or the active species sensor 240, and determine whether the humidity and/or the concentration of the active species contained in the air inside the container 110 is equal to or greater than or less than a reference value. The controller 260 may control the operating time and intensity of one or more of the air filter 120, the air compressor 130, and the plasma generator 140 according to the humidity or the concentration of the active species in the container 110 determined as described above. For example, when determining that the humidity of the air inside the container 110 is equal to or greater than the reference value based on the electrical signal of the humidity sensor 220, the controller 260 may operate the air filter 120 or increase the operating time and/or intensity of the air filter 120. In addition, when determining that the concentration of active species of the air inside the container 110 is equal to or less than the reference value based on the electrical signal of the active species sensor 240, the controller 260 may operate the air compressor 130 and/or the plasma generator 140, or increase the operating time and/or intensity of the air compressor 130 and/or the plasma generator 140.

In an embodiment, the control of the operation of the air filter 120 by the controller 260 as described above, and the control of the operation of the air compressor 130 and/or the plasma generator 140 by the controller 260 may be executed sequentially. For example, the controller 260 may control the operation of the air filter 120 and then control the operation of the air compressor 130 and/or the plasma generator 140, and may also repeat these two steps of control of the operation. In another embodiment, the control of the operation of the air filter 120 by the controller 260 and the control of the operation of the air compressor 130 and/or the plasma generator 140 by the controller 260 may be executed in parallel and simultaneously. In still another embodiment, either the control of the operation of the air filter 120 by the controller 260 or the control of the operation of the air compressor 130 and/or the plasma generator 140 by the controller 260 may be executed.

As described above, by controlling the operation of the air filter 120 by the controller 260 and controlling the operation of the air compressor 130 and/or the plasma generator 140 by the controller 260, the concentration of moisture and active species contained in the air inside the container 110 is maintained within an appropriate range, so that the freshness and preservation period of the cut-flowers 150 may be increased.

FIG. 3 is a perspective view schematically showing a plasma generator 300 according to an embodiment of the present disclosure. The plasma generator 300 shown in FIG. 3 may be used as the plasma generator 140 of the systems 100 and 200 for preserving cut-flowers shown in FIGS. 1 and 2, for example.

In the invention, the plasma generator 300 an atmospheric pressure plasma generator. The plasma generator 300 may include a generator, a high voltage transformer, electrodes for generating plasma discharge, and the like so as to be operated in a normal room temperature/atmospheric pressure environment. Specifically, the plasma generator 300 may include a nozzle part 320 through which plasma is discharged, a gas supply pipe (not shown) where the nozzle part 320 is attached to or detached from one side and a working gas (or compressed air) is supplied from the other side, and a body portion 340 to which a cable or the like connected to a high voltage transformer (not shown) is detached.

In an embodiment, the high frequency high voltage generated by the high voltage transformer is applied to the electrodes installed inside the body portion 340, and a high frequency discharge in the form of an electric arc is generated between the electrodes by the applied voltage. As described above, with the electric arc being generated inside the body portion 340, the working gas contacting the electric arc is converted into the plasma state. The plasma beam generated in the body portion 340 is discharged through an opening of the nozzle part 320.

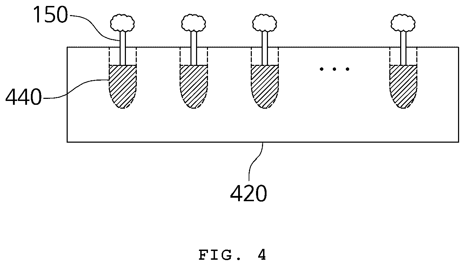

FIG. 4 is a cross-sectional view of a tray 420 for preserving cut-flowers according to an embodiment of the present disclosure. The tray 420 for preserving cut-flowers shown in FIG. 4 may be used as the support 160 installed inside the container 110 shown in FIGS. 1 and 2, for example.

One or more trays 420 for preserving cut-flowers may be disposed in the container (e.g., reference numeral 110 in FIG. 1). The tray 420 for preserving cut-flowers may be configured to mount and preserve the cut-flowers 150. As shown, the tray 420 for preserving cut-flowers may be in a box shape having a rectangular cross section, but is not limited thereto.

In accordance with the invention, the tray 420 for preserving cut-flowers includes a plurality of cut-flower receptacles 440 receiving therein a preservative including nutrients and moisture for maintaining the freshness of the cut-flowers 150. Bacteria may grow in the cut-flower receptacles 440 due to nutrients injected into the preservative for freshness of flowers. The cut-flowers 150 accommodated in the preservative with the propagating bacteria may have a sharp decrease in freshness and preservation period. To solve this problem, the cut-flower receptacles 440 are manufactured with an antibacterial material such as a silver nanomaterial. Specifically, in accordance with the invention, the cut-flower receptacles 440 is molded by injecting a plastic containing a silica nanocomposite containing silver nanoparticles dispersed therein. By accommodating the preservative in the cut-flower receptacles 440 made of antibacterial material, antibacterial and disinfection of the preservative may be enabled.

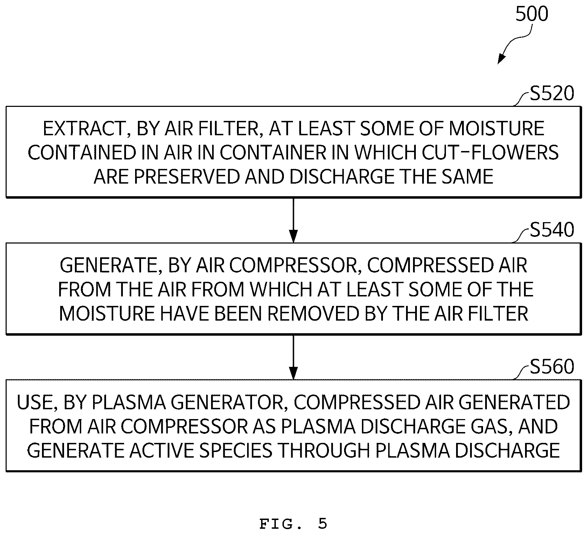

FIG. 5 is a flowchart showing a method 500 for preserving cut-flowers using an atmospheric pressure plasma generator according to an embodiment of the present disclosure.

The method 500 for preserving cut-flowers is initiated by an air filter extracting and discharging at least some of the moisture contained in the air in the container in which the cut-flowers are preserved (S520). In an embodiment, referring to FIG. 1, the air filter 120 may be connected to one side of the container 110 such that air in the container 110 can be introduced into the air filter 120. The air filter 120 is configured to remove at least some of the moisture contained in the air introduced from the container 110. With this configuration, the air in the container 110 may be managed at a constant humidity.

Next, by the air compressor, compressed air is generated from the air from which at least some of the moisture have been removed by the air filter (S540). In accordance with the invention, referring to FIG. 1, the air, from which moisture is removed by the air filter 120, is introduced into the air compressor 130. The air compressor 130 may compress the introduced air to a constant pressure and provide it to the plasma generator 140.

The plasma generator uses the compressed air generated from the air compressor as the plasma discharge gas, and generate active species through plasma discharge (S560). In an embodiment, referring to FIG. 1, the plasma generator 140 generates active species including ozone (O₃) through plasma discharge using the introduced compressed air as the plasma discharge gas. The plasma gas including the active species generated by the plasma generator 140 may be discharged into the container 110 through a nozzle part 142 attached inside the container 110. The harmful bacteria or harmful substances contained in the air inside the container 110 may be removed by the plasma gas including the active species discharged as described above, and accordingly, the freshness or preservation period of the cut-flowers 150 may be increased.

Additionally, in accordance with the invention, it is prepared so that a plurality of cut-flower receptacles are accommodated in the container and the preservative containing nutrients and moisture for maintaining the freshness of the cut-flowers may be accommodated in the cut-flower receptacles. In accordance with the invention, the cut-flower receptacles are molded by injecting a plastic containing a silica nanocomposite containing silver nanoparticles dispersed therein.

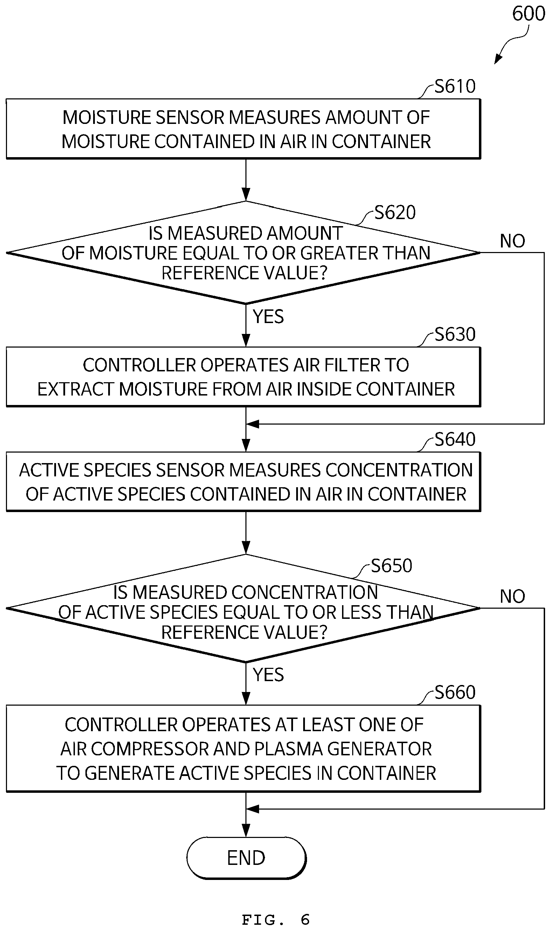

FIG. 6 is a flowchart showing a method 600 for preserving cut-flowers using an atmospheric pressure plasma generator according to another embodiment of the present disclosure.

The method 600 for preserving cut-flowers is initiated by a moisture sensor measuring an amount of moisture contained in the air in the container (S610). In an embodiment, referring to FIG. 2, the humidity sensor 220 installed inside the container 110 may measure the amount of moisture or humidity contained in the air inside the container 110. In addition, the humidity sensor 220 may transmit, to the controller 260, an electrical signal determined according to the amount of moisture or humidity contained in the air inside the container 110.

The controller may determine whether or not the measured amount of moisture is equal to or greater than the reference value (S620). In an embodiment, referring to FIG. 2, the controller 260 may determine whether or not the amount of moisture or humidity of the air inside the container 110 is equal to or greater than the reference value, based on the electrical signal from the humidity sensor 220.

When the amount of moisture measured in step S620 is determined to be equal to or greater than the reference value, the controller may operate the air filter to extract moisture from the air inside the container (S630). In an embodiment, referring to FIG. 2, when determining that the humidity of the air inside the container 110 is equal to or greater than the reference value based on the electrical signal of the humidity sensor 220, the controller 260 may operate the air filter 120 or increase the operating time and/or intensity of the air filter 120. As described above, the air filter 120 may manage the air in the container 110 at a constant humidity by removing at least some of moisture contained in the air introduced from the container 110.

Next, the active species sensor may measure the concentration of the active species contained in the air in the container (S640). In an embodiment, referring to FIG. 2, the active species sensor 240 installed inside the container 110 may transmit, to the controller 260, an electrical signal determined according to the presence or absence of the active species contained in the air inside the container 110 or the concentration of the active species.

The controller may determine whether or not the measured concentration of active species is equal to or less than a reference value (S650). In an embodiment, referring to FIG. 2, the controller 260 may determine whether or not the concentration of the active species in the air inside the container 110 is equal to or less than the reference value based on the electrical signal from the active species sensor 240.

When the concentration of the active species measured in step S650 is determined to be equal to or less than the reference value, the controller may operate at least one of the air compressor and the plasma generator to generate the active species in the container (S660). In an embodiment, referring to FIG. 2, when determining that the concentration of active species of the air inside the container 110 is equal to or less than the reference value based on the electrical signal of the active species sensor 240, the controller 260 may operate the air compressor 130 and/or the plasma generator 140, or increase the operating time and/or intensity of the air compressor 130 and/or the plasma generator 140. The harmful bacteria or harmful substances contained in the air inside the container 110 may be removed by the plasma gas including the active species discharged as described above, and accordingly, the freshness or preservation period of the cut-flowers 150 may be increased.

In an embodiment, the steps (S610 to S630) of controlling the operation of the air filter by the controller as described above and the steps (S640 to S660) of controlling the operation of the air compressor and/or the plasma generator by the controller may be executed sequentially. For example, after executing steps S610 to S630, steps S640 to S660 may be executed, and also, these steps may be repeatedly executed. In another embodiment, the steps (S610 to S630) of controlling the operation of the air filter by the controller and the steps (S640 to S660) of controlling the operation of the air compressor and/or the plasma generator by the controller may be executed in parallel and simultaneously. In still another embodiment, either the steps (S610 to S630) of controlling the operation of the air filter by the controller, or the steps (S640 to S660) of controlling the operation of the air compressor and/or the plasma generator by the controller may be executed.

Additionally, in accordance with the invention, it is prepared so that a plurality of cut-flower receptacles are accommodated in the container and the preservative containing nutrients and moisture for maintaining the freshness of the cut-flowers may be accommodated in the cut-flower receptacles. In accordance with the invention, the cut-flower receptacles are molded by injecting a plastic containing a silica nanocomposite containing silver nanoparticles dispersed therein.

The preferred embodiments of the present invention described above are disclosed for purposes of illustration, and those skilled in the art with ordinary knowledge of the present invention will be able to make various modifications, changes and additions within the scope of the appended set of claims.

## Claims

1. A system (100, 200) for preserving cut-flowers (150) using an atmospheric pressure plasma generator (140), comprising:
a container (110) that preserves cut-flowers (150);
an air filter (120) that extracts at least some of moisture contained in air in the container (110) and discharges said moisture;
an air compressor (130) that generates compressed air from the air from which the at least some of the moisture was removed by the air filter (120); and
a plasma generator (140) that generates active species through plasma discharge using the compressed air generated from the air compressor (130) as a plasma discharge gas,
wherein the system (100) further comprises a receptacle (440) that accommodates the cut-flowers (150) in the container (110), wherein the receptacle (440) is manufactured using an antibacterial material by injection molding a plastic containing a silica nanocomposite containing silver nanoparticles dispersed therein.

2. The system (100, 200) for preserving cut-flowers of claim 1, wherein the plasma generator (140) includes:
a gas supply pipe through which the compressed air is supplied; and
a nozzle part (142) through which plasma gas containing the active species is discharged.

3. The system (200) for preserving cut-flowers of claim 1 or 2, further comprising:
a humidity sensor (220) that measures an amount of moisture contained in the air in the container (110);
an active species sensor (240) that measures a concentration of the active species in the container (110); and
a controller (260) that controls whether or not to operate the air filter (120) based on the amount of moisture measured by the humidity sensor (220), and controls whether or not to operate at least one of the air compressor (130) and the plasma generator (140) based on the concentration of the active species measured by the active species sensor (240).

4. A method (500, 600) for preserving cut-flowers (150) using the system of claim 1, comprising:
extracting, by the air filter (120), at least some of moisture contained in air in the container (110) in which the cut-flowers (150) are preserved and discharging the moisture;
generating, by the air compressor (130), compressed air from the air from which the at least some of the moisture was removed by the air filter (120); and
generating, by the plasma generator (140), active species through plasma discharge by using the compressed air (130) generated from the air compressor (130) as a plasma discharge gas (140).

5. The method (500, 600) for preserving cut-flowers of claim 4, further comprising discharging, by the plasma generator (140), plasma gas containing the active species into the container (110).

6. The method (600) for preserving cut-flowers of claim 4 or 5, further comprising:
measuring, by a humidity sensor (220), an amount of moisture contained in the air in the container (110);
measuring, by an active species sensor (240), a concentration of the active species in the container (110);
controlling, by a controller (260), whether or not to operate the air filter (120) based on the amount of moisture measured by the humidity sensor (220); and
controlling, by the controller (260), whether or not to operate at least one of the air compressor (130) and the plasma generator (140) based on the concentration of active species measured by the active species sensor (240).

## Patentansprüche

1. System (100, 200) zum Konservieren von Schnittblumen (150) unter Verwendung eines Atmosphärendruck-Plasmagenerators (140), umfassend:
einen Behälter (110), der Schnittblumen (150) konserviert;
einen Luftfilter (120), der zumindest einen Teil der in der Luft in dem Behälter (110) enthaltenen Feuchtigkeit extrahiert und die Feuchtigkeit ableitet;
einen Luftkompressor (130), der Druckluft aus der Luft erzeugt, aus der der zumindest ein Teil der Feuchtigkeit durch den Luftfilter (120) entfernt wurde; und
einen Plasmagenerator (140), der aktive Spezies durch Plasmaentladung unter Verwendung der von dem Luftkompressor (130) erzeugten Druckluft als Plasmaentladungsgas erzeugt,
wobei das System (100) ferner ein Behältnis (440) umfasst, das die geschnittenen Blumen (150) in dem Behälter (110) aufnimmt, wobei das Behältnis (440) unter Verwendung eines antibakteriellen Materials durch Spritzgießen eines Kunststoffs hergestellt ist, der ein Siliziumdioxid-Nanokomposit enthält, das darin dispergierte Silber-Nanopartikel enthält.

2. System (100, 200) zum Konservieren von Schnittblumen nach Anspruch 1, wobei der Plasmagenerator (140) umfasst:
ein Gaszufuhrrohr, durch das die Druckluft zugeführt wird; und ein Düsenteil (142), durch das Plasmagas, das die aktive Spezies enthält, abgegeben wird.

3. System (200) zum Konservieren von Schnittblumen nach Anspruch 1 oder 2, das ferner umfasst:
einen Feuchtigkeitssensor (220), der die in der Luft im Behälter (110) enthaltene Feuchtigkeitsmenge misst;
einen Sensor für aktive Spezies (240), der die Konzentration der aktiven Spezies im Behälter (110) misst; und
eine Steuerung (260), die auf der Grundlage der vom Feuchtigkeitssensor (220) gemessenen Feuchtigkeitsmenge steuert, ob der Luftfilter (120) betrieben werden soll oder nicht, und die auf der Grundlage der vom Sensor für aktive Spezies (240) gemessenen Konzentration der aktiven Spezies steuert, ob der Luftkompressor (130) und/oder der Plasmagenerator (140) betrieben werden soll oder nicht.

4. Verfahren (500, 600) zum Konservieren von Schnittblumen (150) unter Verwendung des Systems nach Anspruch 1, umfassend:
Extrahieren von zumindest einem Teil der Feuchtigkeit, die in der Luft in dem Behälter (110) enthalten ist, in dem die Schnittblumen (150) konserviert werden, durch den Luftfilter (120) und Abführen der Feuchtigkeit;
Erzeugen von Druckluft aus der Luft, aus der zumindest ein Teil der Feuchtigkeit durch den Luftfilter (120) entfernt wurde, durch den Luftkompressor (130); und
Erzeugen von aktiven Spezies durch den Plasmagenerator (140) durch Plasmaentladung unter Verwendung der Druckluft (130), die von dem Luftkompressor (130) erzeugt wurde, als ein Plasmaentladungsgas (140).

5. Verfahren (500, 600) zum Konservieren von Schnittblumen nach Anspruch 4, das ferner das Einleiten von Plasmagas, das die aktive Spezies enthält, in den Behälter (110) durch den Plasmagenerator (140) umfasst.

6. Verfahren (600) zum Konservieren von Schnittblumen nach Anspruch 4 oder 5, das ferner umfasst:
Messen einer in der Luft in dem Behälter (110) enthaltenen Feuchtigkeitsmenge durch einen Feuchtigkeitssensor (220);
Messen einer Konzentration der aktiven Spezies in dem Behälter (110) durch einen Sensor für aktive Spezies (240);
Steuern durch eine Steuerung (260), ob der Luftfilter (120) basierend auf der durch den Feuchtigkeitssensor (220) gemessenen Feuchtigkeitsmenge betrieben werden soll oder nicht; und
Steuerung durch die Steuerung (260), ob der Luftkompressor (130) und/oder der Plasmagenerator (140) auf der Grundlage der Konzentration der aktiven Spezies, die durch den Sensor für aktive Spezies (240) gemessen wurde, betrieben wird oder nicht.

## Revendications

1. Système (100, 200) pour conserver des fleurs coupées (150) utilisant un générateur de plasma à pression atmosphérique (140) comprenant :
- un conteneur (110) qui conserve les fleurs coupées (150),
- un filtre à air (120) qui extrait au moins une certaine humidité contenue dans l'air du conteneur (110) et évacue cette humidité,
- un compresseur d'air (130) qui génère de l'air comprimé à partir de l'air dont on a enlevé au moins une certaine partie de l'humidité par le filtre à air (120), et
- un générateur de plasma (140) qui génère des espèces actives par des charges de plasma en utilisant l'air comprimé généré par le compresseur d'air (130) comme gaz de décharge de plasma,
- dans lequel
le système (100) comprend en outre un réceptacle (440) recevant les fleurs coupées (150) dans le conteneur (110),
- le réceptacle (440) étant fabriqué avec une matière antibactérienne par injection d'une matière plastique contenant un nano-composite de silice contenant une dispersion de nanoparticules d'argent.

2. Système (100, 200) pour conserver les fleurs coupées selon la revendication 1,
dans lequel
le générateur de plasma (140) comprend :
- une conduite d'alimentation en gaz par laquelle est fourni l'air comprimé, et
- une buse (142) par laquelle est déchargé le gaz de plasma contenant des espèces actives.

3. Système (200) pour conserver les fleurs coupées selon la revendication 1 ou 2,
comprenant en outre :
- un capteur d'humidité (220) qui mesure le degré d'humidité de l'air dans le container (110),
- un capteur d'espèces actives (240) qui mesure la concentration des espèces actives dans le conteneur (110), et
- un contrôleur (260) qui commande ou non l'activation du filtre à air (120) en se fondant sur le degré d'humidité mesuré par le capteur d'humidité (220) et commande si le compresseur d'air (130) et le générateur de plasma (140) doivent être ou non activés en se fondant sur la concentration des espèces actives, mesurée par le capteur d'espèces actives (240).

4. Procédé (500, 600) pour conserver des fleurs coupées (150) utilisant le système à la revendication 1, consistant à :
- extraire par le filtre à air (120) au moins une certaine partie d'humidité contenue dans l'air du conteneur (110) dans lequel sont conservées les fleurs coupées (150) et évacuer l'humidité,
- générer avec le compresseur d'air (130) de l'air comprimé à partir de l'air dont on a enlevé au moins une partie de l'humidité avec le filtre à air (120), et
- générer, avec le générateur de plasma (140), des espèces actives par des charges de plasma en utilisant l'air comprimé (130) généré par le compresseur d'air (130) comme gaz de décharge de plasma (140).

5. Procédé (500, 600) pour conserver des fleurs coupées selon la revendication 4,
consistant en outre à :
- décharger avec le générateur de plasma (140) le gaz plasma contenu dans les espèces actives du conteneur (110).

6. Procédé (600) pour conserver des fleurs coupées selon la revendication 4 ou 5,
consistant en outre à :
- mesurer avec le capteur d'humidité (220) le degré d'humidité contenu dans l'air du conteneur (110),
- mesurer par le capteur d'espèces actives (240), la concentration en espèces actives d'un conteneur (110),
- contrôler par le contrôleur (260) s'il faut ou non activer le filtre à air (120) en se fondant sur le degré d'humidité mesuré par le capteur d'humidité (220), et
- commander par le contrôleur (260) s'il faut au moins activer le compresseur d'air (130) et le générateur de plasma (140) en se fondant sur la concentration en espèces actives mesurée par le capteur d'espèces actives (240).
